# EUROPEAN PATENT APPLICATION

(11) **EP 4 825 185 A1**
(43) Date of publication of application: **21.10.2026**
(21) Application number: 25472013.9
(22) Date of filing: 16.04.2025
(51) Int. Cl.: A23G 1/40, A23G 1/48, A23G 3/42, A23L 25/00, A23L 33/125, A61K 31/733, A23L 33/21

(54) **NUTRACEUTICAL COMPOSITION OF AN INSULIN-BALANCING SWEET FOOD PRODUCT, A SWEET FOOD PRODUCT WITH SUCH A COMPOSITION AND A METHOD FOR ITS PRODUCTION**

(71) Applicant: MM Miragi 2021 Ltd., 2700 Blagoevgrad (BG)
(72) Inventor: Uzunov, Milena, 2700 Blagoevgrad (BG)
(74) Representative: Benatov, Samuil Gabriel

(57) **Abstract**

The nutraceutical composition of an insulin-balancing sweet food product according to the invention not only does not cause a sharp increase in blood sugar but actively supports its regulation. The composition includes only natural ingredients such as nuts; cocoa products including flavonoid-rich cocoa beans and cocoa butter rich in stearic and oleic acids; a natural sweetener with a glycemic index below 50; and natural thickeners and/or fillers with a low glycemic index and/or additional bioactive ingredients.

## Description

### Field of the Invention

The invention relates to the food industry, more specifically to insulin-balancing foods.

### Prior Art

Traditional sweet food products on the market can be divided into two main categories:
- Conventional - contain sugar, vegetable fats, lecithins, and other additives that trigger insulin spikes and metabolic disorders.
- Healthy products with artificial sweeteners - use sweeteners with a zero glycemic index, but often contain ingredients that are not entirely natural and may have side effects on the body. The replacement of sugar with laboratory-created sweeteners can lead to secondary problems (for example, the body still responds to the sweet taste and may become metabolically confused), as well as to an undesirable aftertaste.

### Summary of the Invention

The objective of the invention is to create a sweet food product and a composition for it that is healthy, free of side effects, and suitable for insulin-sensitive individuals and for those with issues related to blood sugar levels. This objective is achieved by proposing a new type of sweet food product that not only does not cause a sharp increase in blood sugar, but actively supports its balancing.

It supports its balancing. Unlike traditional desserts with high sugar content, this food product is formulated to be suitable for people with insulin resistance and diabetes, as well as for those seeking a healthy alternative. The goal is to achieve a glycemic profile through a selected combination of natural organic ingredients, in which the consumption of the product does not lead to a glucose spike, but rather helps maintain more stable blood sugar levels.

The composition of the sweet food product is a nutraceutical insulin-balancing one, made up of natural ingredients. These natural ingredients are beneficial to the metabolism, have a low glycemic index, and are included in the composition in the following weight ratio:
- 50-60% nuts,
- 20-30% natural cocoa products, including flavonoid-rich cocoa beans and cocoa butter rich in stearic and oleic acids,
- 1-30% natural sweetener with a glycemic index below 50, and
- 0-29% natural thickeners and/or fillers with a low glycemic index and/or additional bioactive ingredients.

In a preferred embodiment of the invention, the natural thickeners and/or fillers with a low glycemic index are at least one of the following: pumpkin flour, roasted or boiled pumpkin purée, zucchini flour, zucchini purée, roasted or boiled carrot purée, roasted or boiled beetroot purée, chickpea purée, coconut flour, almond flour, inulin, water, coconut milk.

In a preferred embodiment of the invention, the additional bioactive ingredients are at least one of the following: berberine, chromium picolinate, white mulberry extract, Gymnema sylvestre, amino acid L-lysine, cinnamon extract, fenugreek extract.

In a preferred embodiment of the invention, the natural sweetener with a glycemic index below 50 is present in an amount of 5 to 10% by weight of the composition.

In a preferred embodiment of the invention, the natural sweetener with a glycemic index below 50 is selected from coconut sugar, palm sugar, powdered dried fruits, yacon syrup, monk fruit extract.

In a preferred embodiment of the invention, the nuts are selected from almonds, walnuts, hazelnuts, pistachios, or a mixture thereof, and are raw or roasted, whole or crushed.

The nutraceutical composition of the insulin-balancing sweet food product according to the invention may be used to reduce postprandial glycemia in a subject in need thereof.

The nutraceutical composition of the insulin-balancing sweet food product according to the invention may be used to improve insulin sensitivity in a subject in need thereof.

The nutraceutical composition of the insulin-balancing sweet food product according to the invention may be used for glycemic control in subjects with insulin resistance.

The sweet food product according to the invention may consist entirely of the nutraceutical composition of the insulin-balancing sweet food product according to the invention.

Preferably, the sweet food product has the following nutritional values per 100 g of product:
2130 kJ - 2350 kJ,
fats 35.0 g - 42.0 g, of which saturated fats 7.9 g - 12.0 g,
carbohydrates 16.5 g - 26.5 g, of which sugars 10.4 g - 21.9 g,
proteins 7.6 g - 10.0 g,
fiber up to 5 g,
salt up to 0.1 g.

The sweet food product according to the invention may be in the form of a bar, or a nut-filled bonbon, or a cocoa drink with coconut milk, or a dessert cream. In another embodiment of the invention, the sweet food product according to the invention may include the nutraceutical composition of the insulin-balancing sweet food product as defined by the invention and additional natural ingredients.

An additional objective of the invention is to create a method for producing such an insulin-balancing sweet food product with a nutraceutical composition as defined in the invention. This objective is achieved by creating a method that includes the following steps:
- Selecting and preparing raw or roasted nuts (with ventilation) at a temperature of 145-150°C for 8-10 minutes,
- Cooling to room temperature,
- Preparing a chocolate base from cocoa products, mixed at a temperature of 45-48°C, then cooled to 38-40°C,
- Mixing a natural sweetener with a glycemic index below 50 with the nuts and the chocolate base,
- Blending the mixture for 30-60 minutes at a temperature of 36-38°C until a homogeneous texture is obtained,
- Tempering the mixture at a temperature of 28-30°C,
- Forming the final product,
- Cooling the final product at a temperature of 10-12°C for 45 minutes.

In a preferred embodiment of the invention, before blending the mixture and after cooling the chocolate base to 38-40°C, natural thickeners and/or fillers with a low glycemic index and/or additional ingredients such as bioactive substances are added at a temperature up to 38°C.

Advantages of the Invention:
- Neutral or positive effect on blood sugar,
- Entirely natural ingredients without artificial sweeteners,
- Supports insulin sensitivity and metabolism,
- Can be consumed by people with diabetes, insulin resistance, or those following a healthy lifestyle.

### Exemplary Embodiment of the Invention

The composition according to the invention, and respectively the sweet food product, contains only natural organic ingredients specifically selected for their effect on insulin sensitivity and blood sugar levels.

The product contains exclusively natural organic ingredients with traceable origin, chosen for their favorable impact on glycemic control.

The term "nuts" in this description refers to all products that are considered nuts in culinary terms, even if botanically they belong to another group-for example, peanuts are legumes, and cashews are seeds, but both are accepted as nuts in culinary use.

A non-exhaustive list of ingredients of the nutraceutical composition of the insulin-balancing sweet food product includes:
- Nuts (approximately 50% of the composition): A mix of nuts such as almonds, walnuts, hazelnuts, pistachios, or others, raw or lightly roasted, whole or crushed. They are rich in fiber and monounsaturated fats, which slow down carbohydrate absorption and protect against sharp insulin spikes. Nuts are slow-digesting and have a low glycemic index, also being rich in proteins and healthy fats. Their consumption slows sugar absorption and prevents abrupt glucose fluctuations. Regular intake of nuts may improve glycemic control and support treatment and the prevention of type 2 diabetes. The polyunsaturated fatty acids in nuts contribute to better insulin sensitivity and help control appetite, while the high content of magnesium and antioxidants in nuts also has an antidiabetic effect. In the composition according to the invention, the nut content provides a significant buffer that slows carbohydrate absorption and delivers sustained energy instead of a sharp spike.
- Cocoa beans: Natural cocoa mass or crushed cocoa beans (e.g., beans with 70%+ cocoa content, as commonly used in dark chocolate). They are rich in flavonoids that improve insulin sensitivity and support glucose metabolism. Cocoa has a low glycemic index (~20) and does not cause sharp blood sugar increases. Cocoa is also a major source of polyphenols, which positively affect metabolism. Regular consumption of cocoa products rich in cocoa flavanols improves insulin sensitivity and may reduce the risk of developing diabetes. The polyphenols in cocoa act as antioxidants, reduce inflammation, and support endothelial (blood vessel) function, which indirectly improves insulin activity. Moreover, cocoa beans-especially those with high cocoa content-have a relatively low glycemic index: the higher the cocoa content, the more fiber and fats and the less sugar. Some types of 100% chocolate have a GI around 23, compared to 60-80 for standard milk chocolate. In the composition according to the invention, cocoa beans not only contribute to the antioxidant profile but also provide an intense flavor, allowing for reduced added sugar.
- Low glycemic sweetener (e.g., coconut sugar, yacon syrup, monk fruit). Unrefined coconut blossom sugar, used in minimal necessary amounts, can provide sweetness. It has a lower glycemic index than white sugar (GI ~35) and contains inulin, which supports blood sugar control and reduces the burden on the pancreas. Coconut sugar is preferred over white sugar due to its significantly lower GI. While standard sugar has a GI of ~60-65, coconut sugar is around GI 35. This means it raises blood glucose more slowly and to a lesser degree, thus lowering the stress on the pancreas and insulin response. The reason is that coconut sugar also contains small amounts of inulin (prebiotic fiber) and is digested more slowly. In combination with other components (nuts, fats), its quantity is limited so that the glycemic effect of the product-for example, a chocolate bar-is minimal. The product contains no refined sugars, glucose-fructose syrups, artificial sweeteners, or synthetic additives.
- Fat-rich ingredients beneficial for balanced nutrition (e.g., cocoa butter and coconut milk):
   - Cocoa butter: The fat extracted from cocoa beans - provides texture and rich flavor. It contains stearic acid, which does not affect insulin levels and supports gradual energy release, and has a neutral effect on cholesterol. Cocoa butter also contains oleic acid. The fats in chocolate are key to slowing glucose absorption-fat presence in food reduces the overall glycemic index of the product by delaying gastric emptying, thus preventing blood sugar spikes.
   - Coconut milk (dry or concentrate): Used for extra creaminess in place of dairy. It is rich in medium-chain triglycerides (MCTs), which are metabolized rapidly and provide energy to the liver without causing glucose spikes. MCTs, in moderate quantities, may even improve insulin sensitivity. Coconut milk is rich in manganese, which helps regulate blood sugar. It contains no lactose or significant carbohydrates, making it safe for diabetics. Additionally, the healthy fats from cocoa and coconut create a sense of satiety, helping control appetite and prevent overeating-an important factor in managing weight and insulin resistance.
   - Additives (optional): Natural bioactive substances with scientifically recognized effects on blood sugar, such as berberine (an alkaloid from herbs like Berberis), cinnamon extract, fenugreek extract, inulin (prebiotic fiber), and others. These bioactive ingredients are added in encapsulated form (e.g., microencapsulation or liposomal systems) to stabilize taste and preserve activity. These additives may be present in low doses sufficient to contribute synergistically to the overall effect.

Example Compositions by Weight Ratio of the Nutraceutical Composition of the Insulin-Balancing Sweet Food Product:
- 50-60% nuts, 20-30% cocoa products including flavonoid-rich cocoa beans and cocoa butter rich in stearic and oleic acids, 1-20% natural sweetener with a glycemic index below 50, and 0% additional ingredients.

50-60% nuts, 20-30% cocoa products including flavonoid-rich cocoa beans and cocoa butter rich in stearic acid and oleic acid, 1-20% natural sweetener with a glycemic index below 50, and from 1 to 29% additional ingredients.

Various additional ingredients may be included in the product without compromising taste, but with the aim of improving glycemic control and metabolism, for example:
- Natural thickeners and fillers with a low glycemic index (GI), offering functional benefits:

### Natural Thickeners and Fillers with Low Glycemic Index

| Ingredient | Form | Characteristics | Benefits |
|---|---|---|---|
| Pumpkin purée | Roasted or boiled, puréed | Moisture, creaminess, fiber | Sweet-neutral flavor; rich in fiber; supports pancreas; stabilizes blood sugar |
| Zucchini purée | Blanched/puréed | Neutral texture and color | Facilitates shaping; contains pectin that lowers blood sugar and improves insulin sensitivity |
| Carrot purée | Roasted/boiled | Light sweetness, beta-carotene | Firms texture |
| Beetroot purée | Boiled or roasted | Earthy taste, color, fiber | Anti-inflammatory properties |
| Chickpea purée | Salt-free, softly boiled | Protein + creaminess | Adds smoothness; neutral taste |
| Coconut flour | Dry powder | Absorbs moisture, fiber | Gluten-free |
| Almond flour | Dry powder | Fat + structure | High in protein |
| Inulin (fiber) | Dry powder | Thickener + microbiome support, prebiotic | Helps sugar absorption |

### Ratio:

The combination of additional ingredients in the form of purées and/or flours can constitute, for example, 10-20% of the total mass, such as:
- For bars: purée + flour in a ratio of ~2:1
- For creams/bonbons: purée ~15-18%, no flour
- For drinks: purée <10%, no flour

The additional ingredients in the form of purées and/or flours facilitate the shaping of the final product, acting as a binding agent.

### Bioactive Ingredients:

- Berberine (encapsulated or with inulin), cinnamon extract, gymnema, L-lysine, insulin-balancing extracts.
- These ingredients are either dissolved in fat or added in powdered form if microgranulated.

### • Berberine:

Berberine is a plant-derived alkaloid extracted from herbs such as *Berberis,* with a well-documented antidiabetic effect.

It activates the AMPK enzyme (adenosine monophosphate-activated protein kinase), which acts as a "metabolic switch" in cells. Through this mechanism, berberine improves insulin sensitivity and lowers blood sugar levels in diabetic and insulin-resistant conditions.

The effect of berberine is comparable to that of metformin - it stimulates cellular glucose uptake and suppresses hepatic glucose output, without requiring the presence of insulin.

It has been proven that in type 2 diabetes, berberine significantly reduces glycated hemoglobin (HbA1c) and fasting blood glucose when taken for 3 months, confirming its antidiabetic effect.

In the context of the product described in the invention, adding a small dose of berberine (e.g., 100-200 mg per serving) would provide an additional direct mechanism for blood sugar control.

The bioactive substance berberine can be integrated into cocoa butter by dissolving and homogenizing the active ingredient before mixing with the dry components.

Berberine may be added via microencapsulation or liposomal encapsulation.

### • Cinnamon:

In addition to berberine, cinnamon is another example of an ingredient that can be included.

It has been proven that cinnamon mimics the effect of insulin and increases insulin sensitivity - meta-analyses report reductions in fasting glucose and HbA1c with cinnamon supplementation.

Cinnamon is known for its ability to improve insulin sensitivity and lower blood sugar levels.

Adding cinnamon to chocolate not only enhances the flavor but also provides additional health benefits.

### • White Mulberry Extract:

The leaves of white mulberry contain compounds that inhibit the enzymes responsible for carbohydrate breakdown, leading to slower sugar absorption and stabilization of blood sugar.

### • Gymnema sylvestre:

This plant is known for its ability to reduce sugar cravings and support blood sugar regulation.

Incorporating gymnema extract into the product may benefit individuals seeking better glucose control.

### • L-Lysine:

This amino acid supports blood sugar balance and may reduce cravings for sweets.

Adding L-lysine to the product composition can enhance its positive effects on glucose metabolism.
- Chromium picolinate:
- Chromium is a mineral that enhances the action of insulin and supports carbohydrate metabolism.
- Including chromium picolinate in the product composition may contribute to improved blood sugar control.

These natural additives are optional and may be the subject of separate product variants (e.g., "chocolate for diabetics with berberine," etc.).

They are not required for the basic formulation to function but provide an additional measurable benefit.

Each of these ingredients individually has a moderate beneficial effect, but their combination in a single product creates synergy.

Nuts and fats reduce the glycemic load of coconut sugar; cocoa polyphenols improve the metabolic profile, making the smaller amount of sugar even more favorably absorbed; herbal extracts like berberine and cinnamon directly support glucose metabolism.

The end result is a composition for a sweet food product that, when consumed, does not lead to a significant increase in blood sugar and, when used regularly, may even contribute to improving long-term glycemic markers.

Based on the composition described in the invention, various chocolate products can be made within this category:
- Chocolate bars with nuts and superfoods
- Nut-filled bonbons
- Cocoa drinks with coconut milk
- Chocolate dessert creams

Based on the composition according to the invention, the following ranges may be defined (which do not limit the scope of protection of the invention) for the main nutritional values per 100 g of product:

### Main Nutritional Values per 100 g of Product

| Category | Minimum Value | Maximum Value |
|---|---|---|
| Energy value | ~2130 kJ (510 kcal) | ~2350 kJ (565 kcal) |
| Fat | 35.0 g | 42.0 g |
| - of which saturated | 7.9 g | 12.0 g |
| Carbohydrates | 16.5 g | 26.5 g |
| - of which sugars | 10.4 g | 21.9 g |
| Proteins | 7.6 g | 10.0 g |
| Fiber (added, if inulin) | up to 5 g | |
| Salt | 0 g - 0.1 g | (naturally occurring) |

The ranges are based on existing products that comply with the basic formulation of our invention.

Depending on whether functional fibers (inulin, etc.) are added or a lower amount of sweetener is used, the values may be adjusted within the indicated limits.

The product with the nutraceutical insulin-balancing composition does not contain added salt-only naturally occurring trace amounts from the ingredients. Carbohydrates and sugars remain at low levels thanks to the coconut sugar, the nut-based composition, and the absence of added sugars.

The consumption of the sweet food product, according to the invention, provides a taste experience comparable to that of high-quality dark chocolate, without the use of refined sugar or artificial sweeteners.

The sweet taste comes from a small amount of coconut sugar and the natural sweetness of the nuts and coconut, enhanced by the aroma of cocoa. This means the consumer makes no compromise in flavor-the sweet food product is pleasant, with a natural caramel note from the coconut sugar and crunchy nuts, contributing to a sensation of pleasure and satiety.

Through careful ingredient selection, the formula eliminates the glycemic spike characteristic of conventional sweet products.

The high content of nuts and cocoa ensures a low effective carbohydrate dose per serving.

The composition, according to the invention, contains no artificial sweeteners or sugar alcohols:
Many "diabetic" products on the market replace sugar with sweeteners like maltitol, sucralose, stevia, etc.

The current formulation deliberately avoids such ingredients. The reasons are:
- Health profile: Artificial sweeteners can mislead the body and trigger metabolic responses undesirable for diabetics.
- Their frequent use can disrupt gut microbiota and paradoxically worsen glycemic control.
- Sugar alcohols (polyols) like maltitol have a lower but still nonzero glycemic index and often cause gastrointestinal discomfort (bloating, diarrhea) in sensitive individuals.
- Taste and aftertaste: Many people report an off-putting aftertaste from artificial sweeteners (e.g., stevia's bitterness or erythritol's cooling effect). The current formulation provides a real sugar flavor, though from a low-GI source and in small quantity.
- Coconut sugar gives a pleasant, brown sugar-like sweetness without any artificial notes.

The nutraceutical composition of the insulin-balancing sweet food product- and accordingly, the sweet food product containing such a composition-may be used for:
- Reducing postprandial glycemia in subjects in need of such support;
- Improving insulin sensitivity in subjects in need thereof;
- Glycemic control in subjects with insulin resistance.

### Production Method - Example Embodiment:

Nuts (e.g., almonds, walnuts, hazelnuts, cashews) may be used raw or roasted. If used raw, the nuts undergo a preparation process in their raw state, which includes visual inspection, cleaning, dehydration at ≤40°C, and microbiological testing to ensure safe use in raw form before being added to the product mixture. This allows for the preservation of all thermosensitive nutrients and enzymes and makes the product suitable for RAW, vegan, or hypoallergenic dietary applications, without compromising the insulin-balancing effect. The flavor of the product with raw nuts is more neutral and naturally nutty.

If roasted, the nuts are lightly roasted at 145-150°C for no more than 10 minutes with active ventilation.

They are then cooled to room temperature and stabilized by natural dehydration. The use of lower temperature and short roasting time compared to standard nut roasting methods preserves fatty acids (oleic, linoleic), proteins, and antioxidants.

It avoids the formation of acrylamide and other heat-induced toxins that occur in traditional roasting above 160°C.

This achieves a richer flavor compared to the raw variant, with aroma development and slight caramelization, without compromising the nutritional value of the nuts.

Cocoa mass (20-30%) and cocoa butter (5-8%) are mixed at a maximum temperature of 45-48°C until fully liquefied, then cooled to 38-40°C. A natural low-GI sweetener-e.g., coconut sugar, yacon syrup, or monk fruit-is added, and the mixture is blended to full homogeneity. Classical chocolate tempering is avoided at this stage

Mixing at 55-60°C would result in the loss of flavonoids and antioxidants in the cocoa. The temperature range is selected specifically to preserve the nutraceutical and antioxidant properties of the cocoa beans and fats. The use of natural sweeteners with a glycemic index <5 further avoids increasing the glycemic load, making the chocolate base functional for people with insulin resistance.

After cooling to no more than 38-40°C, functional ingredients with proven insulin-balancing effects are added to the base:
- Berberine (in microencapsulated or powdered form);
- Inulin (plant fiber);
- Gymnema sylvestre, cinnamon, chromium picolinate, fenugreek extract, etc.

These ingredients are thermosensitive-high temperatures would destroy their active molecules. Adding them at ≤38°C ensures the preservation of the biological activity of the nutraceuticals. This combination of functional ingredients creates a unique synergistic effect for blood sugar balancing-without the use of artificial sweeteners or medications.

At this phase, natural thickeners/structuring agents can also be added, such as purée of pumpkin, beetroot, zucchini, chickpeas, or coconut and almond flour. All ingredients are homogenized at 36-38°C. The resulting mixture is formed into bars, bonbons, creams, or beverages, depending on the consistency.

The method does not include thermal pasteurization or stabilizers, which is why structure is achieved through natural purées and fibers.

Full bioactivity of the included nutraceuticals, fats, and antioxidants is maintained.

It allows flexibility in the final product forms without losing functionality.

Each phase is conducted under strictly controlled low temperatures, which is key to preserving the nutraceutical quality of the product.

A combination of natural ingredients with functional activity is used, which remain bioactive thanks to the processing method.

The method excludes additives, artificial stabilizers, and emulsifiers, achieving the desired structure using only natural means.

The result is a sweet product that not only does not raise blood sugar but helps regulate it, aligning with the concept of functional food/nutraceutical.

For specialists in the field, it will be clear that various modifications of the nutraceutical composition of the insulin-balancing sweet food product are possible, and these also fall within the scope of the invention as defined in the appended claims.

All parts of the invention may be replaced with technically equivalent features. The reference numbers of the technical features are included in the claims solely for clarity and thus have no limiting effect on the interpretation of the elements referenced by these numbers.

## Claims

1. A nutraceutical composition of an insulin-balancing sweet food product, composed of natural ingredients, **characterized in that** the natural ingredients are metabolically beneficial ingredients with a low glycemic index and include by weight ratio:
- 50-60% nuts,
- 20-30% natural cocoa products, including flavonoid-rich cocoa beans and cocoa butter rich in stearic and oleic acids,
- 1-30% natural sweetener with a glycemic index below 50, and
- 0-29% natural thickeners and/or fillers with a low glycemic index and/or additional bioactive ingredients.

2. The composition according to claim 1, **characterized in that** the natural thickeners and/or fillers with a low glycemic index are at least one of the following: pumpkin flour, roasted or boiled pumpkin purée, zucchini flour, zucchini purée, roasted or boiled carrot purée, roasted or boiled beetroot purée, chickpea purée, coconut flour, almond flour, inulin, water, coconut milk.

3. The composition according to claim 1, **characterized in that** the additional bioactive ingredients are at least one of the following: berberine, chromium picolinate, white mulberry extract, Gymnema sylvestre, amino acid L-lysine, cinnamon extract, fenugreek extract.

4. The composition according to any of the preceding claims, **characterized in that** the natural sweetener with a glycemic index below 50 is present in an amount from 5 to 10% by weight of the composition.

5. The composition according to any of the preceding claims, **characterized in that** the natural sweetener with a glycemic index below 50 is selected from coconut sugar, palm sugar, powdered dried fruits, yacon syrup, monk fruit extract.

6. The composition according to any of the preceding claims, **characterized in that** the nuts are selected from almonds, walnuts, hazelnuts, pistachios, or a mixture thereof, and are raw or roasted, whole or crushed.

7. The composition according to any of the preceding claims for use in reducing postprandial glycemia in a subject in need thereof.

8. The composition according to any of claims 1-6 for use in improving insulin sensitivity in a subject in need thereof.

9. The composition according to any of claims 1-6 for use in glycemic control in subjects with insulin resistance.

10. A sweet food product composed entirely of the nutraceutical composition of an insulin-balancing sweet food product according to any of claims 1-6.

11. A sweet food product made entirely from the nutraceutical composition of an insulin-balancing sweet food product according to any of claims 1-6, having a nutritional value per 100 g of product as follows:
2130 kJ - 2350 kJ,
fats 35.0 g - 42.0 g, of which saturated fats 7.9 g - 12.0 g,
carbohydrates 16.5 g - 26.5 g, of which sugars 10.4 g - 21.9 g,
proteins 7.6 g - 10.0 g,
fiber up to 5 g,
salt up to 0.1 g.

12. A sweet food product made entirely from the nutraceutical composition of an insulin-balancing sweet food product according to any of claims 1-6, wherein the sweet food product is in the form of a bar, or a bonbon with nut filling, or a cocoa drink with coconut milk, or a dessert cream.

13. A sweet food product including the nutraceutical composition of an insulin-balancing sweet food product according to any of claims 1-6 and additional natural ingredients.

14. A method for producing an insulin-balancing sweet food product according to any of claims 10-13 with a nutraceutical composition as defined in any of claims 1-6, comprising the steps of:
- selecting and preparing raw or roasted nuts with ventilation at a temperature of 145-150°C for 8-10 minutes,
- cooling to room temperature,
- preparing a chocolate base from cocoa products, mixed at a temperature of 45-48°C and then cooled to 38-40°C,
- mixing a natural sweetener with glycemic index below 50 with the nuts and the chocolate base,
- blending the mixture for 30-60 minutes at a temperature of 36-38°C until a homogeneous texture is achieved,
- tempering the mixture at a temperature of 28-30°C,
- forming the final product,
- cooling the final product at a temperature of 10-12°C for 45 minutes.

15. A method for producing an insulin-balancing sweet food product with a nutraceutical composition according to claim 14, **characterized in that** before blending the mixture and after cooling the chocolate base to 38-40°C, natural thickeners and/or fillers with a low glycemic index and/or additional ingredients such as bioactive substances are added at a temperature up to 38°C.
